(19) Europäisches Patentamt / European Patent Office / Office européen des brevets

(11) **EP 3 978 136 B1**

(12) **EUROPEAN PATENT SPECIFICATION**

(45) Date of publication and mention
of the grant of the patent:
**10.05.2023  Bulletin 2023/19**

(21) Application number: **20200087.3**

(22) Date of filing: **05.10.2020**

(51) International Patent Classification (IPC):
**B04B 11/02** *(2006.01)*     **B04B 7/08** *(2006.01)*
**B04B 7/12** *(2006.01)*

(52) Cooperative Patent Classification (CPC):
**B04B 11/02; B04B 7/08; B04B 7/12**

(54) **MODULAR CENTRIFUGAL SEPARATOR SYSTEM**

MODULARES ZENTRIFUGALABSCHEIDERSYSTEM

SYSTÈME MODULAIRE DE SÉPARATEUR CENTRIFUGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.04.2022  Bulletin 2022/14**

(73) Proprietor: **Alfa Laval Corporate AB
221 00 Lund (SE)**

(72) Inventor: **THORWID, Peter
172 66 SUNDBYBERG (SE)**

(74) Representative: **Alfa Laval Attorneys
Alfa Laval Corporate AB
Patent Department
P.O. Box 73
221 00 Lund (SE)**

(56) References cited:
EP-A1- 3 666 394     WO-A1-2015/181177
US-A1- 2011 319 248     US-B2- 6 973 925

## Description

TECHNICAL FIELD

The invention relates to a set of exchangeable separation inserts for a modular centrifugal separator system, to a modular centrifugal separator system, and to a method for operating a modular centrifugal separator system.

BACKGROUND

In the field of pharmaceuticals, biopharmaceuticals, biotechnology and thereto related fields, separation of substances from a liquid mixture, such as separation of cells from a cell culture mixture, are performed in a sterile environment. Traditionally, equipment made e.g. from stainless steel has been used, which equipment is sterilised between batches.

Disposable separation equipment made for single use, i.e. for one batch or a limited number of batches, has been suggested. For instance, US2011/0319248 discloses a single use centrifuge and WO 2015/181177 discloses a separator comprising an exchangeable inner drum.

Such disposable separation equipment is supplied to the user in a sterile manner. Thus, a sterile environment for the product in the separator may be provided without sterilisation of the separation equipment at the production facility of the user.

EP 3666394 discloses a base unit and a modular centrifugal separator. The base unit comprises a stationary frame, a rotatable member, and a drive unit. The rotatable member delimits an inner space being configured for receiving at least one part of an exchangeable separation insert therein. The rotatable member is provided with a first opening at a first axial end configured for a first fluid connection of the exchangeable separation insert to extend through the first opening. The rotatable member comprises a second opening at a second axial end configured for a second fluid connection of the exchangeable separation insert to extend through the second opening.

US 6973925 discloses a ventilation device for a crankcase of an internal combustion engine. Said device comprises a centrifugal oil separator comprising plates/disks, which has an inlet for an oil-air mixture, an air vent for the purified air and an oil outlet for the oil. In this context of a gas liquid separator, the use of different numbers of plates adapted to the volumetric flow of gas requiring oil removal is described.

Different separation processes may require different separation capacities.

SUMMARY

It would be advantageous to achieve different separation capacities in the context of a modular centrifugal separator system with a base unit and exchangeable separation inserts. In particular, it would be desirable, within the limitations set by a particularly sized base unit of a modular centrifugal separator system, to provide different separation capacities. To better address one or more of these concerns, at least one of a set of exchangeable separation inserts for a modular centrifugal separator system, a modular centrifugal separator system, and a method for operating a modular centrifugal separator system, having the features defined in the independent claims is provided.

According to an aspect of the invention, there is provided a set of exchangeable separation inserts for a modular centrifugal separator system comprising a base unit. The set of exchangeable separation inserts comprises at least a first exchangeable separation insert and a second exchangeable separation insert. Each of the first and second exchangeable separation inserts comprises a rotor casing configured to rotate about an axis of rotation and forming a separation space, frustoconical separation discs arranged in the separation space, and fluid connections for a liquid feed mixture, a separated heavy phase, and a separated light phase. The frustoconical separation discs of the first exchangeable separation insert have a first area equivalent and the frustoconical separation discs of the second exchangeable separation insert have a second area equivalent. Each of the rotor casings of the first and second separation inserts have a same external shape to an extent that they fit inside an inner space of a rotatable member of a base unit of a modular centrifugal separator system and abut against at least part of an inner surface of the rotatable member, the inner surface delimiting at least part of the inner space. The first area equivalent differs from the second area equivalent with each of the first and second area equivalents calculated for the same rotational speed.

According to a further aspect of the invention, there is provided a modular centrifugal separator system configured for separating a liquid feed mixture into a heavy phase and light phase. The modular centrifugal separator system comprises a base unit and a first exchangeable separation insert. The base unit comprises a stationary frame, a rotatable member configured to rotate about an axis of rotation arranged in the stationary frame, and a drive unit for rotating the rotatable member about the axis of rotation. The rotatable member delimits an inner space at least in a radial direction, the inner space being configured for receiving at least one part of the first exchangeable separation insert therein. The modular separation system comprises a set of exchangeable separation inserts according to any one of aspects and/or embodiments discussed herein. The first exchangeable separation insert forms part of the set of exchangeable separation inserts.

According to a further aspect of the invention, there is provided a method for operating a modular centrifugal separator system configured for separating a liquid feed mixture into a heavy phase and light phase according to any one of aspects and/or embodiments discussed herein. The method comprises steps of:

- providing the first exchangeable separation insert,
- mounting the first exchangeable separation insert in the inner space of the rotatable member,
- separating a first batch of liquid feed mixture in the modular centrifugal separator system into a first heavy phase and a first light phase utilising the first exchangeable separation insert,
- removing the first exchangeable separation insert from the inner space of the rotatable member,
- providing the second exchangeable separation insert,
- mounting the second exchangeable separation insert in the inner space of the rotatable member, and
- separating a second batch of liquid feed mixture in the modular centrifugal separator system into a second heavy phase and a second light phase utilising the second exchangeable separation insert.

Since the set of exchangeable separation inserts of the modular centrifugal separator system comprises first and second exchangeable separation inserts have different area equivalents at the same rotational speed, different separation capacities are provided by each of the set of exchangeable separation inserts, the modular centrifugal separator system, and the method. Thus, within the limitations of one and the same base unit of the modular centrifugal separator system, by using either the first or second exchangeable separation insert, an adequate separation capacity is selectable.

The inventor has contemplated the situation; that for certain separation operations, centrifugation of certain liquid feed mixtures, their contents may not be suited to be subjected to centrifugal forces above a certain level, such as e.g. a liquid feed mixture comprising a fermentation broth containing CHO cells. The inventor has realised that different separation capacities can be achieved in a modular centrifugal separator comprising an exchangeable separation insert at up to a maximum separation speed, i.e. at up to a maximum centrifugal force, defined by properties of a particular liquid feed mixture but with the use of different total surfaces areas provided in the exchangeable separation inserts. Thus, in the context of disposable separation equipment made for single use, i.e. for separating one batch only or a limited number of batches, the supplying of the first and second exchangeable separation inserts discussed herein provide adaptation to required or desired separation capacity in the modular centrifugal separator system.

In this manner also a cost reduction for a lower capacity separation may be provided by the use of a separation insert having fewer separation discs or smaller diameter separation discs.

In the context of traditional disc stack centrifugal separators configured for continuous or multiple use and which are devised for being disassembled only for service and/or cleaning purposes, differently sized separators having different separation capacities are well known. Also, the use of differently sized disc stacks utilising the same type and size of separation discs are known, such as e.g. discussed in the above cited US 6973925.

However, the provision of differently sized disc stacks of separation discs of the same type and size has traditionally served the purpose of providing centrifugal separators in different versions. In the selection of a suitable centrifugal separator or version of centrifugal separator for a particular separation operation, inter alia the type of liquid feed mixture, its volumetric flow, viscosity, temperature, feed composition, and particle content determine the selection of one particular centrifugal separator. Once selected, the composition of different parts and in particular the number and size of separation disc is not altered.

According to the present invention the exchangeability of exchangeable separation inserts of a modular centrifugal separator is utilised for providing differing separation performance within one and the same base unit. Thus, a greater flexibility in the use of a modular centrifugal separator, now forming part of a modular centrifugal separator system including first and second exchangeable separation inserts, is utilised. This greater flexibility may be utilised e.g. in the context of separating different cell culture mixtures, particular biomolecules produced, or particular cells produced.

The term separation performance relates to flow of liquid feed mixture that can be separated to a certain concentration of heavy phase and/or a certain clarity of light phase.

A modular centrifugal separator may comprise two main parts, the base unit and an exchangeable separation insert. In such a modular centrifugal separator separation of a liquid feed mixture may be performed. The modular centrifugal separator system may comprise such a modular centrifugal separator and a further exchangeable separation insert, i.e. comprising a set of exchangeable separation inserts as defined above.

Herein, descriptions may refer to a modular centrifugal separator. Such descriptions also relate to a modular centrifugal separator system and its different components. In particular, general aspects of the exchangeable separation insert of the modular centrifugal separator relate to both the first and second exchangeable separation insert of the set of exchangeable separation inserts and of the modular centrifugal separator system.

The base unit may comprise basic components for supporting and rotating the exchangeable separation insert. The exchangeable separation insert may be configured for the actual separation of the liquid feed mixture to take place in the separation space thereof. The liquid feed mixture may flow through one fluid connection into the separation space and the separated heavy and light phases may leave the separation space via one fluid connection each.

The term area equivalent, of the frustoconical separation discs of the first and second exchangeable separation inserts is a measure of the separation capacity of the relevant exchangeable separation insert. That is, the higher the area equivalent, the higher the separation performance of an exchangeable separation insert.

The area equivalent, Ae, is calculated according to the following formula:

$$\text{Ae} = (2\pi/3g)\,(\text{Ncot}\alpha\omega^2(r_y^3 - r_i^3))$$

Wherein: g = gravity, N = the number of frustoconical separation discs of the separation insert, $\alpha$ = half the apex angle of the frustoconical separation discs, i.e. the angle between the axis of rotation and an inner surface of one of the frustoconical separation discs, $\omega$ = rotational speed of the separation insert, $r_y$ = outer radius of the frustoconical separation disc, $r_i$ = inner radius of the frustoconical separation disc.

In order to provide a comparative measure between the first and second exchangeable separation inserts when not in use, i.e. non-rotating, a rotational speed neutral figure is provided by the area equivalent of the respective first and second exchangeable separation insert calculated for the same rotational speed, e.g. $\omega$ = 1 for both the first and second exchangeable separation insert.

The exchangeable separation insert may be configured for single use, i.e. for separation of one batch only or a limited number of batches of liquid feed mixture. The base unit on the other hand may be configured for repeated use with different exchangeable separation inserts such as the first and second exchangeable separation inserts, i.e. the base unit may be used for the separation of numerous batches of liquid feed mixture using different exchangeable separation inserts.

The first and second exchangeable separation inserts may be configured to form the only part of the modular centrifugal separator system, which is in contact with the liquid feed mixture, and the separated heavy and light phases. Thus, the first and second exchangeable separation inserts may be provided to a user as a sterile entity. The sterile entity may include parts configured for separating the liquid feed mixture as well as conduits for the liquid feed mixture and the separated heavy and light phases. The exchangeable separation inserts are mounted in the base unit by the user. Thus, the user will readily have available a centrifugal separator with a sterile environment for separation of the liquid feed mixture.

The rotatable member of the base unit may be rotatably supported in the stationary frame. The rotatable member may be supported in the stationary frame without the aid of a spindle or other kind of rotor shaft. The stationary frame is stationary in the sense that it is stationary during use of modular centrifugal separator.

According to embodiments, the first area equivalent differing from the second area equivalent may be provided by a difference in number of frustoconical separation discs between the first and second exchangeable separation insert. In this manner, different separation performance may be provided by differing number of frustoconical separation discs in the first and second exchangeable separation discs.

According to embodiments, the frustoconical separation discs of the first exchangeable separation insert and the frustoconical separation discs of the second exchangeable separation insert may have a same total height along the axis of rotation. A distance between at least two of the frustoconical separation discs of the first exchangeable separation insert may differ from a distance between at least two of the frustoconical separation discs of the second exchangeable separation insert. In this manner, the frustoconical separation discs may occupy the same amount of space in the separation space in the first and second exchangeable separation discs, while the distance between the separation discs in the respective first and second exchangeable separation insert differ to accommodate for the differing number of separation discs in the first and second exchangeable separation insert.

According to embodiments, the first area equivalent differing from the second area equivalent may be provided by at least one of:

- a difference in outer diameter of the frustoconical separation discs between the first and second exchangeable separation insert,
- a difference in inner diameter of the frustoconical separation discs between the first and second exchangeable separation insert, and/or
- a difference in an angle between the axis of rotation of the first and second exchangeable separation insert and an inner surface of one of the frustoconical separation discs between the first and second exchangeable separation insert. In this manner, further or additional alternatives may be given for providing different separation performances in the first and second exchangeable separation inserts.

According to embodiments, the rotor casing of each of the first and second exchangeable separation insert has a first axial end portion and a second axial end portion and each of the first and second exchangeable separation insert may comprise at least a first fluid connection arranged at the first axial end portion. In this manner, the liquid feed mixture may be led to the exchangeable separation inserts or one of the heavy phase or light phase may be led from the exchangeable separation inserts at the first axial end portion thereof. Optionally, a second and even a third fluid connection may be provided at the first axial end portion permitting one or more of the liquid feed mixture, the heavy phase, and/or light phase to also enter or exit the exchangeable separation insert at the first axial end portion thereof.

In connection with such embodiments of the set of exchangeable separation inserts, according to embodiments of the modular centrifugal separator system, the rotatable member has a first axial end and a second axial end. The rotatable member may be provided with a first opening at the first axial end configured for at least a first fluid connection of the first or second exchangeable separation insert to extend through the first opening. In this manner, the liquid feed mixture may be led to the exchangeable separation inserts or one of the heavy phase or light phase may be led from the exchangeable separation inserts via the first opening of the rotatable member. Optionally, a second and even a third fluid connection of the exchangeable separation inserts may extend to/from the exchangeable separation inserts via the first opening of the rotatable member.

According to embodiments, each of the first and second exchangeable separation insert may comprise at least a second fluid connection arranged at the second axial end portion. In this manner, the liquid feed mixture may be led to the exchangeable separation inserts or one of the heavy phase or light phase may be led from the exchangeable separation inserts at the second axial end portion thereof. A third fluid connection may be provided at the first or second axial end portion.

In connection with such embodiments of the set of exchangeable separation inserts, according to embodiments of the modular centrifugal separator system, the rotatable member may comprises a second opening at the second axial end configured for at least a second fluid connection of the first or second exchangeable separation insert to extend through the second opening. In this manner, the liquid feed mixture may be led to the exchangeable separation inserts or one of the heavy phase or light phase may be led from the exchangeable separation inserts via the second opening of the rotatable member. A third fluid connection of the exchangeable separation inserts may extend through the first or second opening of the rotatable member.

According to embodiments of the method, each of the first and second batch of liquid feed mixture may comprise solid matter. The first batch of liquid feed mixture may differ from the second batch of liquid feed mixture by at least one of: type of solid matter, concentration of solid matter, and/or content of the separated first light phase and content of the separated second light phase.

According to embodiments of the method, each of the first and second batch of liquid feed mixture may be a cell culture mixture. The cell culture mixture may be produced as a fermentation broth in a fermenter tank. The heavy phase separated in the centrifugal separator system may comprise a concentrate of cells in a small portion of fermentation broth. The light phase separated in the centrifugal separator system may contain substantially only fermentation broth.

Further features of, and advantages with, the invention will become apparent when studying the appended claims and the following detailed description.

BRIEF DESCRIPTION OF THE DRAWINGS

Various aspects and/or embodiments of the invention, including its particular features and advantages, will be readily understood from the example embodiments discussed in the following detailed description and the accompanying drawings, in which:

> Fig. 1 schematically illustrates a modular centrifugal separator system according to embodiments,
> Fig. 2 schematically illustrates a cross section through a base unit of a modular centrifugal separator and modular centrifugal separator system,
> Fig. 3 schematically illustrates a cross-section through an exchangeable separation insert according to embodiments,
> Fig. 4 schematically illustrates a cross section through a portion of a modular centrifugal separator, and
> Fig. 5 illustrates a method for operating a modular centrifugal separator system.

DETAILED DESCRIPTION

Aspects and/or embodiments of the invention will now be described more fully. Like numbers refer to like elements throughout. Well-known functions or constructions will not necessarily be described in detail for brevity and/or clarity.

The disclosure of EP 3666394 is enclosed herein by reference. In particular, details of the modular separator, its base unit and exchangeable separation insert are applicable in the context of the present invention including the set of exchangeable separation inserts, the modular centrifugal separator system, and the method for operating a modular centrifugal separator system.

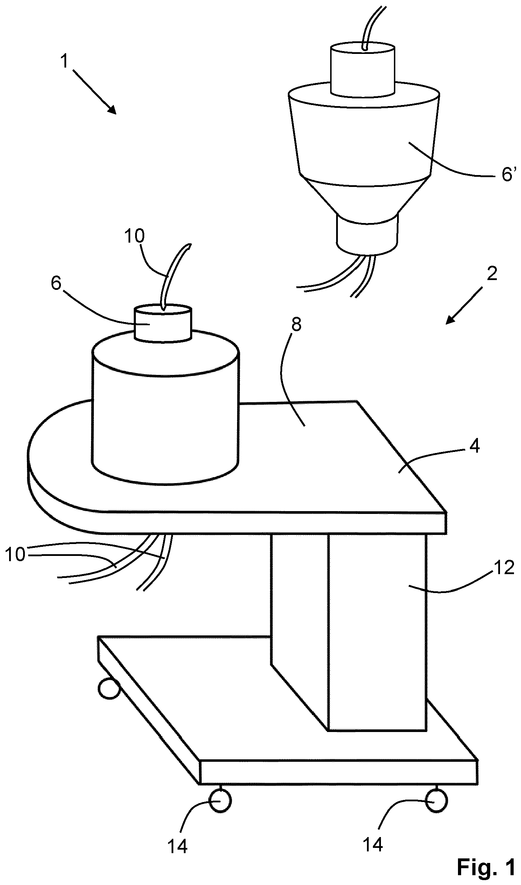

**Fig. 1** schematically illustrates a modular centrifugal separator system 1 according to embodiments. The modular centrifugal separator system 1 comprises a base unit 4 and a first exchangeable separation insert 6 and a second exchangeable separation insert 6'. The modular centrifugal separator system 1 may be configured for use in the field of pharmaceuticals, biopharmaceuticals, and/or biotechnology. The modular centrifugal separator system 1 may form part of a set-up in a plant for the production of cells, such as CHO cells (Chinese Hamster Ovary cells) or other matter resulting from processes in the biotech industry.

Herein the base unit 4 with one of the first or second exchangeable separation inserts 6, 6' mounted there may be referred to as a modular centrifugal separator 2.

The modular centrifugal separator 2 is configured for separating a liquid feed mixture into a heavy phase and light phase. For instance, the liquid feed mixture may be formed by a fermentation broth including a cell culture, the heavy phase may comprise the cells separated from the main part of the fermentation broth. The light phase may be formed by main part the fermentation broth without the cells or with only a minimum rest amount of cells.

The modular centrifugal separator system 1 is modular in the sense that it comprises the base unit 4 and the first and second exchangeable separation inserts 6, 6'. The exchangeable separation inserts 6, 6' are exchanged for each new batch of liquid feed mixture, which is to be separated. Alternatively, the exchangeable separation inserts 6, 6' may be exchanged for each new type of liquid feed mixture, which is to be separated, i.e. subsequent batches containing same type of liquid feed mixtures may be separated with the same exchangeable separation insert 6, 6'.

During use of the modular centrifugal separator system 1 the liquid feed mixture, the heavy phase, and the light phase only come into contact with the relevant first or second exchangeable separation insert 6, 6' of the modular centrifugal separator system 1. Naturally, conduits in the form of tubes 10 configured for conducting the liquid feed mixture to the exchangeable separation insert 6 and for conducting the heavy phase and the light phase from the exchangeable separation insert 6 also come into contact with the liquid feed mixture and the heavy and light phases. The tubes 10 may form part of the exchangeable separation insert 6. The base unit 4 does not come into contact with the liquid feed mixture or any of the heavy and light phases.

The first and second exchangeable separation inserts 6, 6' form a set of exchangeable separation inserts of the modular centrifugal separator system 1.

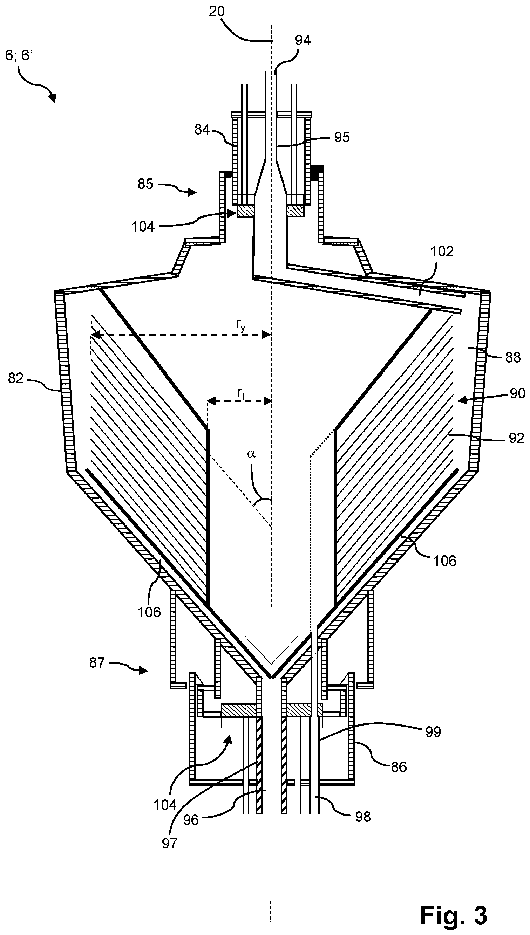

The first and second exchangeable separation inserts 6, 6' are further discussed below with reference to **Fig. 3.**

The base unit 4 comprises components for supporting and rotating the exchangeable separation insert. Thus, the base unit 4 comprises inter alia a stationary frame 8, a rotatable member, and a drive unit for rotating the rotatable member. The stationary frame 8 comprises a vertical member 12. Part of the drive unit may be arranged in the vertical member 12.

The stationary frame 8 is stationary during use of the modular centrifugal separator. However, the base unit 4 as such may be movable, e.g. in order to be positioned at different locations at a production facility of the user. For this purpose, the stationary frame 8 may be provided with wheels 14.

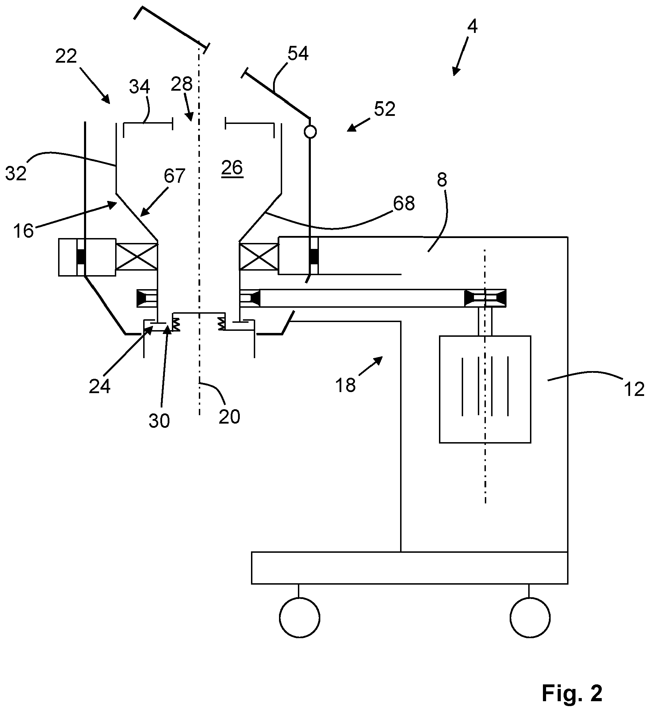

The base unit 4 is further discussed below with reference to **Fig. 2**

**Fig. 2** schematically illustrates a cross section through the base unit 4 of the modular centrifugal separator 2 and the modular centrifugal separator system 1 of **Fig. 1.** That is, in **Fig. 2** exchangeable separation inserts have been omitted.

As mentioned above, the base unit 4 comprises the stationary frame 8, the rotatable member 16, and the drive unit 18. The rotatable member 16 is arranged in the stationary frame 8 and is configured to rotate about an axis 20 of rotation. The drive unit 18 is configured for rotating the rotatable member 16 about the axis 20 of rotation.

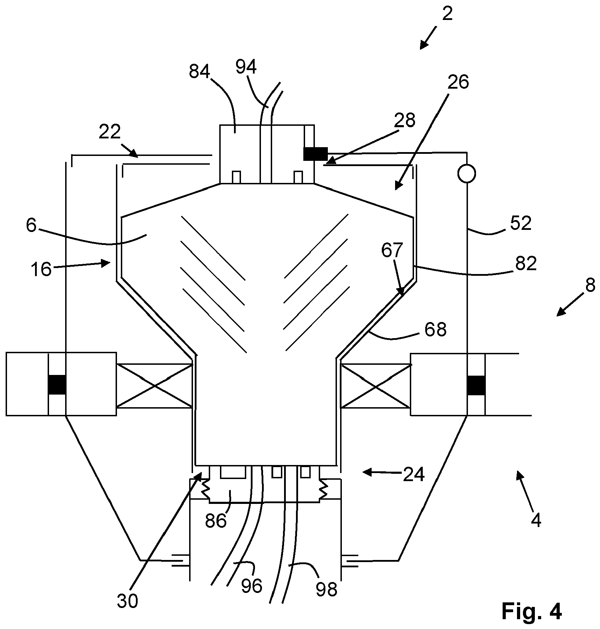

Seen along the axis 20 of rotation, the rotatable member 16 has a first axial end 22 and a second axial end 24. The rotatable member 16 delimits an inner space 26 at least in a radial direction. The radial direction extends perpendicularly to the axis 20 of rotation. The inner space 26 is configured for receiving at least one part of the first or second exchangeable separation insert therein, see further below with reference to **Figs. 3 and 4.**

The rotatable member 16 is provided with a first opening 28 at the first axial end 22. The rotatable member 16 further is provided with a second opening 30 at the second axial end 24. Each of the first and second openings 28, 30 forms a through hole in the rotatable member 16. Thus, the inner space 26 is accessible via each of the first and second openings 28, 30. Accordingly, the first and second openings 28, 30 are configured for fluid connections of the first or second exchangeable separation insert to extend therethrough. See further below with reference to **Figs. 3 and 4.** According to alternative embodiments, the rotatable member 16 may be provided with only the first opening 28, in which case all fluid connections of the first or second exchangeable separation insert extend through the first opening 28 only.

Access to the inner space 26 is provided via a cap 34. Thus, the first or second exchangeable separation insert may be mounted in and removed from the inner space 26. The rotatable member 16 is arranged inside a stationary housing 52. The housing 52 comprises a lid 54. In an open position of the lid 54, access is provided to the rotatable member 16 inside the housing 52, e.g. for exchange of the exchangeable separation insert.

The inner space 26 of the rotatable member 16 is delimited at least in part by an inner surface 67. The first and second separation inserts have a same external shape to an extent that they fit inside the inner space 26. Each of the first and second separation insert, when fitted in the inner space 26 abut against at least part of an inner surface 67. Thus, the first and second exchangeable separation inserts are supported in the inner space 26.

In the illustrated embodiments, the rotatable member 16 comprises a frustoconical wall member 68 having an imaginary apex in a region of the second axial end 24. The frustoconical wall member 68 delimits a portion of the inner space 26 and comprises the inner surface 67. When positioned in the inner space 26, an exchangeable separation insert having a conical or frustoconical shape may be supported by the frustoconical wall member 68.

**Fig. 3** schematically illustrates a cross-section through an exchangeable separation insert 6, 6' according to embodiments. The exchangeable separation insert 6 may form part of a modular centrifugal separator system, such as the modular centrifugal separator system 1 discussed above in connection with **Fig. 1.** Accordingly, the illustrated exchangeable separation insert 6, 6' exemplifies both the first exchangeable separation insert 6 and the second exchangeable separation insert 6' discussed above with reference to **Fig. 1.** Accordingly, the following discussion relates both to the first and second exchangeable separation insert 6, 6' as well as to the set of exchangeable separation inserts. Aspects, by which the first and second exchangeable separation inserts 6, 6' differ from each other, are specifically discussed.

The exchangeable separation insert 6, 6' is configured for part of it to be arranged inside an inner space 26 of a rotatable member 16 of a base unit, e.g. a base unit 4 as discussed above in connection with **Fig. 2.**

The exchangeable separation insert 6, 6' comprises a rotor casing 82, a first stationary portion 84 arranged at a first end portion 85 of the rotor casing 82, and a second stationary portion 86 arranged at a second end portion 87 of the rotor casing 82. The exchangeable separation insert 6 is configured to rotate about an axis 20 of rotation. The rotor casing 82 is arranged between the first stationary portion 84 and the second stationary portion 86. During operation of the modular centrifugal separator, the first stationary portion 84 is arranged at an upper axial end of the exchangeable separation insert 6, whereas the second stationary portion 86 is arranged at a lower axial end of the exchangeable separation insert 6.

Each of the rotor casings 82 of the first and second separation inserts 6, 6' have a same external shape to an extent that they fit inside an inner space 26 of a rotatable member 16 of a base unit 4 of a modular centrifugal separator system 1 and abut against at least part of an inner surface 67 of the rotatable member, e.g. as discussed above with reference to **Fig. 2.**

The rotor casing 82 delimits a separation space 88 therein. The exchangeable separation insert 6 comprises a stack 90 of frustoconical separation discs 92 arranged in the separation space 88. The separation discs 92 in the stack 90 are arranged with an imaginary apex at the second stationary portion 86, and/or pointing towards the second stationary portion 86. The stack 90 may comprise at least 50 separation discs 92, such as at least 100 separation discs 92, such as at least 150 separation discs 92. Mentioned as an example, a separation disc 92 may have an outer radius, $r_y$, within a range of -80 - 200 mm, an inner radius, $r_i$, within a range of 30 - 50 mm, and an angle $\alpha$ between the axis 20 of rotation and an inner surface of the disc 92 (half the apex angle of the frustoconical separation discs 92) within a range of 35 - 45 degrees. For clarity reasons, only a few discs 92 are shown in **Fig. 3.**

In the illustrated embodiments, each of the first and second exchangeable separation insert 6, 6' comprises at least a first fluid connection 94 arranged at the first axial end portion 85. Each of the first and second exchangeable separation insert 6, 6' comprises at least a second fluid connection 96 arranged at the second axial end portion 87.

More specifically, the exchangeable separation insert 6, 6' comprises a first fluid connection 94 arranged at the first stationary portion 84. A first conduit portion 95 forms part of the first fluid connection 94. The first conduit portion 95 of the first fluid connection 94 extents through the first stationary portion 84. The exchangeable separation insert 6 comprises a second fluid connection 96 arranged at the second stationary portion 86. A second conduit portion 97 forms part of the second fluid connection 96. The second conduit portion 97 of the second fluid connection 96 extents through the second stationary portion 86. In these embodiments, the exchangeable separation insert 6 comprises a third fluid connection 98 arranged at the second stationary portion 86. A third conduit portion 99 forms part of the third fluid connection 98. The third conduit portion 99 of the third fluid connection 98 extents through the second stationary portion 86. Inside the rotor casing 82 there is arranged one or more outlet conduits 102 for the separated heavy phase from the separation space 88. The first, second, and third fluid connections 94, 96, 98 may comprise tubing, such as plastic tubing.

The first stationary portion 84 abuts against the rotor casing 82. The second stationary portion 86 abuts against the rotor casing 82. Seals 104 are provided between the respective first and second stationary portions 84, 86 and the rotor casing 82. The seals 104 form mechanical seals between the stationary portions 84, 86 and the rotor casing 82. Thus, the exchangeable separation insert 6 is provided with mechanically hermetically sealed inlet and outlets. A mechanical hermetical seal includes an abutment between part of the rotatable rotor casing and a stationary portion.

During operation, the exchangeable separation insert 6, 6', arranged in a rotatable member 16, is brought into rotation around the axis 20 of rotation. Liquid feed mixture to be separated is supplied via the second fluid connection 96 arranged in the second stationary portion 86 and guiding channels 106 into the separation space 88. The liquid feed mixture to be separated is guided along an axially upwardly path into the separation space 88. Due to a density difference the liquid feed mixture is separated into a liquid light phase and a liquid heavy phase. This separation is facilitated by the interspaces between the separation discs 92 of the stack 90 fitted in the separation space 88. The heavy phase may comprise particles, such as e.g. cells. The heavy phase may comprise a concentrated mixture of light phase and particles.

The separated liquid heavy phase is collected from the periphery of the separation space 88 via outlet conduit 102 and is forced out of the rotor casing 82 to the first fluid connection 94 arranged in the first stationary portion 84. Separated liquid light phase is forced radially inwardly through the stack 90 of separation discs 92 and led out of the rotor casing 82 to the third fluid connection 98 arranged in the second stationary portion 86. Consequently, in this embodiment, the liquid feed mixture is supplied at a lower axial end of the exchangeable separation insert 6, the separated light phase is discharged at the lower axial end, and the separated heavy phase is discharged at the upper axial end of the exchangeable separation insert 6.

The first and second exchangeable separation inserts 6, 6' differ from each other with respect to aspects of the frustoconical separation discs 92. More specifically, the frustoconical separation discs 92 of the first exchangeable separation insert 6 have a first area equivalent and the frustoconical separation discs 92 of the second exchangeable separation insert 6' have a second area equivalent. The first area equivalent differs from the second area equivalent with each of the first and second area equivalents calculated for the same rotational speed. Thus, different separation performances are provided in the first and second exchangeable separation inserts 6, 6'.

According to embodiments, the first area equivalent differing from the second area equivalent is provided by a difference in number of frustoconical separation discs 92 between the first and second exchangeable separation insert 6, 6'. Thus, the stacks 90 of separation discs 92 of the first and second exchangeable separation inserts 6, 6' may have different hights along the axis 20 of rotation.

According to alternative embodiments, the stacks 90 of separation discs 92 of the first and second exchangeable separation inserts 6, 6' may have the same hights, but still with a difference in number of frustoconical separation discs 92. Accordingly, the frustoconical separation discs 92 of the first exchangeable separation insert 6 and the frustoconical separation discs 92 of the second exchangeable separation insert 6' may have a same total height along the axis 20 of rotation. With a distance between at least two of the frustoconical separation discs 92 of the first exchangeable separation insert 6 differing from a distance between at least two of the frustoconical separation discs 92 of the second exchangeable separation insert 6', the same height of both stacks 90 of separation discs may be achieved. The difference in distance between the separation discs 92 of the first and second exchangeable separation inserts 6, 6' may be achieved by a difference in the height of caulks or other distance members, such as pointed projections, provided on the surfaces of the separation discs 92.

According to embodiments, the first area equivalent differing from the second area equivalent is alternatively or further provided by at least one of:

- a difference in outer diameter of the frustoconical separation discs 92 between the first and second exchangeable separation insert 6, 6',
- a difference in inner diameter of the frustoconical separation discs 92 between the first and second exchangeable separation insert 6, 6', and/or
- a difference in the angle $\alpha$ between the axis 20 of rotation and an inner surface of one of the frustoconical separation discs 92 between the first and second exchangeable separation insert 6, 6', i.e. a difference in the conicity of the separation discs 92.

According to alternative embodiments, the stack 90 of frustoconical separation discs 92 may be arranged with an imaginary apex at the first stationary portion 84, and/or pointing towards the first stationary portion 84.

According to alternative embodiments, the first, second, and third fluid connections and corresponding fluid pathways inside the rotor casing 82 and the separation space 88 may be arranged differently than in the illustrate embodiments, e.g. two fluid connections may be provided at the first stationary portion 84 and one fluid connection at the second stationary portion 86.

According to alternative embodiments, the exchangeable separation insert 6, 6' may comprise only the first or second stationary portion 84, 86. In such embodiments, the first, second, and third fluid connections are provided at the first or second stationary portion 84, 86, respectively.

**Fig. 4** schematically illustrates a cross section through a portion of a modular centrifugal separator 2. More specifically, **Fig. 4** shows a cross section through a housing 52, a rotatable member 16, and an exchangeable separation insert 6, 6' of the modular centrifugal separator 2. The modular centrifugal separator 2 forms part of a modular centrifugal separator system 1 as discussed above in connection with **Figs. 1 and 2.** The exchangeable separation insert 6 may be a first or second exchangeable separation insert 6, 6' as discussed above in connection with **Figs. 1** - **3.** Accordingly, in the following, reference is also made to **Figs. 1** - **3.**

In **Fig. 4** the exchangeable separation insert 6 is shown mounted in the base unit 4. Part of the exchangeable separation insert 6 is received in the inner space 26 of the rotatable member 16.

The inner space 26 of the rotatable member 16 is delimited at least in part by an inner surface 67. The inner space 26 is configured for receiving at least one part of the first and second exchangeable separation inserts 6, 6', respectively, therein.

The first and second separation inserts 6, 6' have a same external shape to an extent that they fit inside the inner space 26. Each of the first and second separation insert, when fitted in the inner space 26 abut against at least part of an inner surface 67. Thus, the first and second exchangeable separation inserts 6, 6' are supported in the inner space 26.

For instance, and as also mentioned above with reference to **Fig. 2,** the rotatable member 16 comprises a frustoconical wall member 68 which forms part of the inner surface 67. A portion of the first or second exchangeable separation insert 6, 6' has a conical or frustoconical shape. The conical or frustoconical portion of the exchangeable separation insert 6, 6' is supported by the frustoconical wall member 68.

It is to be noted that the first and second exchangeable separation inserts 6, 6' need not be supported in exactly the same manner in the inner space 26. Each the first and second exchangeable separation inserts 6, 6' may be supported in a different manner by the inner surface 67. For instance, one of the exchangeable separation inserts 6, 6' may be supported at larger diameter portion of the frustoconical wall member 67 and the other of the exchangeable separation inserts 6, 6' may be supported at smaller diameter portion of the frustoconical wall member 67.

During use of the modular centrifugal separator 2, the first stationary portion 84 is fixed in relation to the stationary frame 8 and the second stationary portion 86 is fixed in relation to the stationary frame 8.

In the illustrated embodiments, the rotatable member 16 has a first axial end 22 and a second axial end, and the rotatable member 16 is provided with a first opening 28 at the first axial end 22 configured for at least a first fluid connection 94 of the first or second exchangeable separation insert 6, 6' to extend through the first opening 28. In this manner, the first fluid connection 94 may be arranged for extending into or out of the rotatable member 16.

In the illustrated embodiments, the rotatable member 16 comprises a second opening 30 at the second axial end 24 configured for at least a second fluid connection 96 of the first or second exchangeable separation insert 6, 6' to extend through the second opening 30. In this manner, the second fluid connection 96 may be arranged for extending into or out of the rotatable member 16.

In the illustrated embodiments, a third fluid connection 98 of the first or second exchangeable separation insert 6, 6' may extend through the second opening 30.

According to alternative embodiments, the third fluid connection 98 of the first or second exchangeable separation insert 6, 6' may extend through the first opening 28.

According to further alternative embodiments, only the first or second opening 28, 30 is provided in the rotatable member and all fluid connections of the first or second exchangeable separation insert 6, 6' extend through the first or second opening 28, 30 respectively.

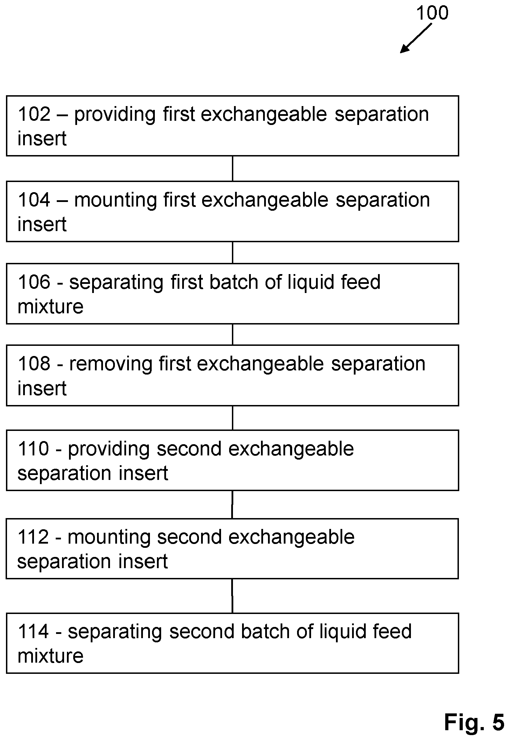

Fig. 5 illustrates a method 100 for operating a modular centrifugal separator system configured for separating a liquid feed mixture into a heavy phase and light phase. The modular centrifugal separator system may be a modular centrifugal separator system 1 as discussed above in connection with **Figs. 1-4.** Accordingly, in the flowing reference is also made to **Figs. 1-4.**

The method 100 comprises steps of:

- providing 102 the first exchangeable separation insert 6,
- mounting 104 the first exchangeable separation insert 6 in the inner space 26 of the rotatable member 16,
- separating 106 a first batch of liquid feed mixture in the modular centrifugal separator system 1 into a first heavy phase and a first light phase utilising the first exchangeable separation insert,
- removing 108 the first exchangeable separation insert 6 from the inner space 26 of the rotatable member 16,
- providing 110 the second exchangeable separation insert 6',
- mounting 112 the second exchangeable separation insert 6' in the inner space 26 of the rotatable member 16,
- separating 114 a second batch of liquid feed mixture in the modular centrifugal separator system 1 into a second heavy phase and a second light phase utilising the second exchangeable separation insert 6'.

According to embodiments, the liquid feed mixture may be a cell culture mixture, i.e. each of the first and second batch of liquid feed mixture may be a cell culture mixture. The first batch of liquid feed mixture in the form of a cell culture mixture may be produced in a fermentation broth in a fermenter tank. Accordingly, a fermentation period in the fermenter tank may be followed by separation of the first batch of liquid feed mixture in the modular centrifugal separator system 1 utilising the first exchangeable separation insert 6. That is, the fermentation period may be followed by steps 102 - 108 of the method 100 being performed. The second batch of liquid feed mixture, after a subsequent or parallel fermentation period in a fermenter tank, may be follow in the modular centrifugal separator system 1 utilising the second exchangeable separation insert 6'. That this, the subsequent or parallel fermentation period may be followed by steps 110- 114 of the method 100 being performed.

According to embodiments, each of the first and second batch of liquid feed mixture may comprise solid matter. The first batch of liquid feed mixture may differ from the second batch of liquid feed mixture by at least one of: type of solid matter, concentration of solid matter, and/or content of the separated first light phase and content of the separated second light phase.

In embodiments wherein the liquid feed mixture is a cell culture mixture, the solid matter corresponds to the cells of the cell culture mixture. The light phase is the liquid of the cell culture mixture with cells removed. The heavy phase contains the separated cells and a small amount of liquid.

The fermentation in the fermenter tank may for example be for expression of an extracellular biomolecule, such as an antibody, from a mammalian cell culture mixture. The extracellular biomolecule may be extracted from the separated light phase in subsequent process step/s. In other processes the cells of the cell culture mixture may be, or may contain, the sought-after substance from the fermentation in the fermenter tank. The cells or the substance may be extracted from the separated heavy phase in subsequent process step/s.

Thus, the first batch of liquid feed mixture differing from the second batch of liquid feed mixture by type of solid matter may be by different types of cells of the first and second cell culture mixtures. The first batch of liquid feed mixture differing from the second batch of liquid feed mixture by concentration of solid matter may be by different concentrations of cells, of the same or different kinds. The first batch of liquid feed mixture differing from the second batch of liquid feed mixture by content of the separated first light phase and content of the separated second light phase may be by a difference in extracellular biomolecule.

According to embodiments, following the separating step 114, the second exchangeable separation insert 6' may be removed from the inner space 26 of the rotatable member 16, and a further first exchangeable separation insert 6 or a further second exchangeable separation insert 6' may be provided and mounted in the inner space 26. Alternatively, further kinds of exchangeable separation inserts with further differing separation capacities may be provided and mounted in the inner space 26.

It is to be understood that the foregoing is illustrative of various example embodiments and that the invention is defined only by the appended claims. A person skilled in the art will realize that the example embodiments may be modified, and that different features of the example embodiments may be combined to create embodiments other than those described herein, without departing from the scope of the invention, as defined by the appended claims.

**Claims**

1. A set of exchangeable separation inserts (6, 6') for a modular centrifugal separator system (1) comprising a base unit (4), the set comprising at least a first exchangeable separation insert (6) and a second exchangeable separation insert (6'), wherein

   each of the first and second exchangeable separation inserts (6, 6') comprises a rotor casing (82) configured to rotate about an axis of rotation (20) and forming a separation space (88), frustoconical separation discs (92) arranged in the separation space (88), and fluid connections (10, 94, 96, 98) for a liquid feed mixture, a separated heavy phase, and a separated light phase, wherein
   the frustoconical separation discs (92) of the first exchangeable separation insert (6) have a first area equivalent and the frustoconical separation discs (92) of the second exchangeable separation insert (6') have a second area equivalent, wherein
   each of the rotor casings (82) of the first and second separation inserts (6, 6') have a same external shape to an extent that they fit inside an inner space (26) of a rotatable member (16) of a base unit (4) of a modular centrifugal separator system (1) and abut against at least part of an inner surface (67) of the rotatable member (16), the inner surface (67) delimiting at least part of the inner space (26),
   **characterised in that**
   the first area equivalent differs from the second area equivalent with each of the first and second area equivalents calculated for the same rotational speed.

2. The set according to claim 1, wherein the first area equivalent differing from the second area equivalent is provided by a difference in number of frustoconical separation discs (92) between the first and second exchangeable separation insert (6, 6').

3. The set according to claim 2, wherein the frustoconical separation discs (92) of the first exchangeable separation insert (6) and the frustoconical separation discs (92) of the second exchangeable separation insert (6') have a same total height along the axis (20) of rotation, and wherein a distance between at least two of the frustoconical separation discs (92) of the first exchangeable separation insert (6) differs from a distance between at least two of the frustoconical separation discs (92) of the second exchangeable separation insert (6').

4. The set according to any one of the preceding claims, wherein the first area equivalent differing from the second area equivalent is provided by at least one of:

   - a difference in outer diameter of the frustoconical separation discs (92) between the first and second exchangeable separation insert (6, 6'),
   - a difference in inner diameter of the frustoconical separation discs (92) between the first and second exchangeable separation insert (6, 6'), and/or
   - a difference in an angle $\alpha$ between an axis 20 of rotation of the first and second exchangeable separation insert (6, 6') and an inner surface of one of the frustoconical separation discs (92) between the first and second exchangeable separation insert (6, 6').

5. The set according to any one of the preceding claims, wherein the rotor casing (82) of each of the first and second exchangeable separation insert (6, 6') has a first axial end portion (85) and a second axial end portion (87), and wherein each of the first and second exchangeable separation insert (6, 6') comprises at least a first fluid connection (94) arranged at the first axial end portion (85).

6. The set according to claim 5, wherein each of the first and second exchangeable separation insert (6, 6') comprises at least a second fluid connection (96) arranged at the second axial end portion (87).

7. A modular centrifugal separator system (1) configured for separating a liquid feed mixture into a heavy phase and light phase, the modular centrifugal separator system comprising a base unit (4) and a first exchangeable separation insert (6), wherein

    the base unit (4) comprises a stationary frame (8), a rotatable member (16) configured to rotate about an axis (20) of rotation arranged in the stationary frame (8), and a drive unit (18) for rotating the rotatable member (16) about the axis (20) of rotation, wherein
    the rotatable member (16) delimits an inner space (26) at least in a radial direction, the inner space (26) being configured for receiving at least one part of the first exchangeable separation insert (6) therein,
    **characterised in that**
    the modular separation system comprises (1) a set of exchangeable separation inserts (6, 6') according to any one of the preceding claims, wherein
    the first exchangeable separation insert (6) forms part of the set of exchangeable separation inserts (6, 6').

8. The modular centrifugal separator system (1) according to claim 7, wherein the rotatable member (16) has a first axial end (22) and a second axial end (24), and the rotatable member (16) is provided with a first opening (28) at the first axial end (22) configured for at least a first fluid connection (94) of the first or second exchangeable separation insert (6, 6') to extend through the first opening (28).

9. The modular centrifugal separator system (1) according to claim 8, wherein, the rotatable member (16) comprises a second opening (30) at the second axial end (24) configured for at least a second fluid connection (96) of the first or second exchangeable separation insert (6, 6') to extend through the second opening (30).

10. A method (100) for operating a modular centrifugal separator system (1) configured for separating a liquid feed mixture into a heavy phase and light phase according to any one of claims -7 - 9, comprising steps of:

    - providing (102) the first exchangeable separation insert (6),
    - mounting (104) the first exchangeable separation insert (6) in the inner space (26) of the rotatable member (16),

    - separating (106) a first batch of liquid feed mixture in the modular centrifugal separator system (1) into a first heavy phase and a first light phase utilising the first exchangeable separation insert,
    - removing (108) the first exchangeable separation insert (6) from the inner space (26) of the rotatable member (16),
    - providing (110) the second exchangeable separation insert (6'),
    - mounting (112) the second exchangeable separation insert (6') in the inner space (26) of the rotatable member (16),
    - separating (114) a second batch of liquid feed mixture in the modular centrifugal separator system (1) into a second heavy phase and a second light phase utilising the second exchangeable separation insert (6').

11. The method according to claim 10, wherein each of the first and second batch of liquid feed mixture comprises solid matter, and wherein the first batch of liquid feed mixture differs from the second batch of liquid feed mixture by at least one of:

    - type of solid matter,
    - concentration of solid matter, and/or
    - content of the separated first light phase and content of the separated second light phase.

12. The method according to claim 10 or 11, wherein each of the first and second batch of liquid feed mixture is a cell culture mixture.


**Patentansprüche**

1. Satz von austauschbaren Abscheidungseinsätzen (6, 6') für ein eine Basiseinheit (4) umfassendes modulares Zentrifugalabscheidersystem (1), wobei der Satz mindestens einen ersten austauschbaren Abscheidungseinsatz (6) und einen zweiten austauschbaren Abscheidungseinsatz (6') umfasst, wobei

    sowohl der erste als auch der zweite austauschbare Abscheidungseinsatz (6, 6') ein zum Drehen um eine Drehachse (20) konfiguriertes und einen Abscheidungsraum (88) bildendes Rotorgehäuse (82), im Abscheidungsraum (88) angeordnete kegelstumpfförmige Abscheidungsscheiben (92) und Fluidverbindungen (10, 94, 96, 98) für ein flüssiges Beschickungsgemisch, eine abgeschiedene schwere Phase und eine abgeschiedene leichte Phase umfasst, wobei die kegelstumpfförmigen Abscheidungsscheiben (92) des ersten austauschbaren Abscheidungseinsatzes (6) ein erstes Flächenäquivalent und die kegelstumpfförmigen Abscheidungsscheiben (92) des zweiten austauschbaren Abscheidungseinsatzes (6') ein zweites Flächenäquivalent aufweisen, wobei

die Rotorgehäuse (82) sowohl des ersten als auch des zweiten Abscheidungseinsatzes (6, 6') eine gleiche Außengestalt in einem solchen Ausmaß aufweisen, dass sie in einen Innenraum (26) eines drehbaren Elements (16) einer Basiseinheit (4) eines modularen Zentrifugalabscheidersystems (1) passen und an mindestens einem Teil einer Innenfläche (67) des drehbaren Elements (16) anliegen, wobei die Innenfläche (67) mindestens einen Teil des Innenraums (26) begrenzt,

**dadurch gekennzeichnet, dass**

das erste Flächenäquivalent sich vom zweiten Flächenäquivalent unterscheidet, wobei sowohl das erste als auch das zweite Flächenäquivalent für dieselbe Drehgeschwindigkeit berechnet wird.

2.  Satz nach Anspruch 1, wobei das erste Flächenäquivalent, das sich vom zweiten Flächenäquivalent unterscheidet, durch eine Differenz in einer Anzahl von kegelstumpfförmigen Abscheidungsscheiben (92) zwischen dem ersten und zweiten austauschbaren Abscheidungseinsatz (6, 6') bereitgestellt ist.

3.  Satz nach Anspruch 2, wobei die kegelstumpfförmigen Abscheidungsscheiben (92) des ersten austauschbaren Abscheidungseinsatzes (6) und die kegelstumpfförmigen Abscheidungsscheiben (92) des zweiten austauschbaren Abscheidungseinsatzes (6') eine gleiche Gesamthöhe entlang der Drehachse (20) aufweisen, und wobei sich ein Abstand zwischen mindestens zwei der kegelstumpfförmigen Abscheidungsscheiben (92) des ersten austauschbaren Abscheidungseinsatzes (6) von einem Abstand zwischen mindestens zwei der kegelstumpfförmigen Abscheidungsscheiben (92) des zweiten austauschbaren Abscheidungseinsatzes (6') unterscheidet.

4.  Satz nach einem der vorhergehenden Ansprüche, wobei das sich vom zweiten Flächenäquivalent unterscheidende erste Flächenäquivalent bereitgestellt ist durch mindestens eine von Folgendem:

    - eine Differenz in einem Außendurchmesser der kegelstumpfförmigen Abscheidungsscheiben (92) zwischen dem ersten und zweiten austauschbaren Abscheidungseinsatz (6, 6'),
    - eine Differenz in einem Innendurchmesser der kegelstumpfförmigen Abscheidungsscheiben (92) zwischen dem ersten und zweiten austauschbaren Abscheidungseinsatz (6, 6'), und/oder
    - eine Differenz in einem Winkel $\alpha$ zwischen einer Drehachse 20 des ersten und zweiten austauschbaren Abscheidungseinsatzes (6, 6') und einer Innenfläche von einer der kegelstumpfförmigen Abscheidungsscheiben (92) zwischen dem ersten und zweiten austauschbaren Abscheidungseinsatz (6, 6').

5.  Satz nach einem der vorhergehenden Ansprüche, wobei das Rotorgehäuse (82) sowohl des ersten als auch des zweiten austauschbaren Abscheidungseinsatzes (6, 6') einen ersten axialen Endabschnitt (85) und einen zweiten axialen Endabschnitt (87) aufweist, und wobei sowohl der erste als auch der zweite austauschbare Abscheidungseinsatz (6, 6') mindestens eine am ersten axialen Endabschnitt (85) angeordnete erste Fluidverbindung (94) umfasst.

6.  Satz nach Anspruch 5, wobei sowohl der erste als auch der zweite austauschbare Abscheidungseinsatz (6, 6') mindestens eine am zweiten axialen Endabschnitt (87) angeordnete zweite Fluidverbindung (96) umfasst.

7.  Modulares Zentrifugalabscheidersystem (1), das zum Abscheiden eines flüssigen Beschickungsgemischs in eine schwere Phase und eine leichte Phase konfiguriert ist, wobei das modulare Zentrifugalabscheidersystem eine Basiseinheit (4) und einen ersten austauschbaren Abscheidungseinsatz (6) umfasst, wobei

    die Basiseinheit (4) einen stationären Rahmen (8), ein drehbares Element (16), das zum Drehen um eine in dem stationären Rahmen (8) angeordnete Drehachse (20) konfiguriert ist, und eine Antriebseinheit (18) zum Drehen des drehbaren Elements (16) um die Drehachse (20) umfasst, wobei

    das drehbare Element (16) einen Innenraum (26) mindestens in einer radialen Richtung begrenzt, wobei der Innenraum (26) zum Aufnehmen mindestens eines Teils des ersten austauschbaren Abscheidungseinsatzes (6) darin konfiguriert ist,

    **dadurch gekennzeichnet, dass**

    das modulare Abscheidungssystem (1) einen Satz von austauschbaren Abscheidungseinsätzen (6, 6') nach einem der vorhergehenden Ansprüche umfasst, wobei

    der erste austauschbare Abscheidungseinsatz (6) Teil des Satzes von austauschbaren Abscheidungseinsätzen (6, 6') ist.

8.  Modulares Zentrifugalabscheidersystem (1) nach Anspruch 7, wobei das drehbare Element (16) ein erstes axiales Ende (22) und ein zweites axiales Ende (24) aufweist, und das drehbare Element (16) mit einer ersten Öffnung (28) am ersten axialen Ende (22) versehen ist, die so konfiguriert ist, dass sich mindestens eine erste Fluidverbindung (94) des ersten oder zweiten austauschbaren Abscheidungseinsatzes (6, 6') durch die erste Öffnung (28) erstreckt.

9. Modulares Zentrifugalabscheidersystem (1) nach Anspruch 8, wobei das drehbare Element (16) eine zweite Öffnung (30) am zweiten axialen Ende (24) umfasst, die so konfiguriert ist, dass sich mindestens eine zweite Fluidverbindung (96) des ersten oder zweiten austauschbaren Abscheidungseinsatzes (6, 6') durch die zweite Öffnung (30) erstreckt.

10. Verfahren (100) zum Betreiben eines modularen Zentrifugalabscheidersystems (1), konfiguriert zum Abscheiden eines flüssigen Beschickungsgemischs in eine schwere Phase und eine leichte Phase nach einem der Ansprüche 7 bis 9, das die folgenden Schritte umfasst:

    - Bereitstellen (102) des ersten austauschbaren Abscheidungseinsatzes (6),
    - Montieren (104) des ersten austauschbaren Abscheidungseinsatzes (6) im Innenraum (26) des drehbaren Elements (16),
    - Abscheiden (106) einer ersten Charge an flüssigem Beschickungsgemisch in dem modularen Zentrifugalabscheidersystem (1) in eine erste schwere Phase und eine erste leichte Phase unter Verwendung des ersten austauschbaren Abscheidungseinsatzes,
    - Entfernen (108) des ersten austauschbaren Abscheidungseinsatzes (6) aus dem Innenraum (26) des drehbaren Elements (16),
    - Bereitstellen (110) des zweiten austauschbaren Abscheidungseinsatzes (6'),
    - Montieren (112) des zweiten austauschbaren Abscheidungseinsatzes (6') im Innenraum (26) des drehbaren Elements (16),
    - Abscheiden (114) einer zweiten Charge an flüssigem Beschickungsgemisch in dem modularen Zentrifugalabscheidersystem (1) in eine zweite schwere Phase und eine zweite leichte Phase unter Verwendung des zweiten austauschbaren Abscheidungseinsatzes (6').

11. Verfahren nach Anspruch 10, wobei sowohl die erste als auch die zweite Charge an flüssigem Beschickungsgemisch Feststoff umfasst, und wobei sich die erste Charge an flüssigem Beschickungsgemisch von der zweiten Charge an flüssigem Beschickungsgemisch unterscheidet durch mindestens eines von Folgendem:

    - Typ von Feststoff,
    - Konzentration von Feststoff, und/oder
    - Gehalt der abgeschiedenen ersten leichten Phase und Gehalt der abgeschiedenen zweiten leichten Phase.

12. Verfahren nach Anspruch 10 oder 11, wobei sowohl die erste als auch die zweite Charge an flüssigem Beschickungsgemisch ein Zellkulturgemisch ist.

**Revendications**

1. Ensemble d'inserts de séparation échangeables (6, 6') pour un système modulaire de séparateur centrifuge (1) comprenant une unité de base (4), l'ensemble comprenant au moins un premier insert de séparation échangeable (6) et un deuxième insert de séparation échangeable (6'), dans lequel

    chacun des premier et deuxième inserts de séparation échangeables (6, 6') comprend un carter de rotor (82) configuré pour tourner autour d'un axe de rotation (20) et formant un espace de séparation (88), des disques de séparation tronconiques (92) agencés dans l'espace de séparation (88), et des raccordements fluidiques (10, 94, 96, 98) pour un mélange d'alimentation liquide, une phase lourde séparée, et une phase légère séparée, dans lequel
    les disques de séparation tronconiques (92) du premier insert de séparation échangeable (6) ont un premier équivalent de surface et les disques de séparation tronconiques (92) du deuxième insert de séparation échangeable (6') ont un deuxième équivalent de surface, dans lequel
    chacun des carters de rotor (82) des premier et deuxième inserts de séparation (6, 6') ont une forme externe semblable dans la mesure où ils s'insèrent dans un espace interne (26) d'un élément rotatif (16) d'une unité de base (4) d'un système modulaire de séparateur centrifuge (1) et butent contre au moins une partie d'une surface interne (67) de l'élément rotatif (16), la surface interne (67) délimitant au moins une partie de l'espace interne (26),
    **caractérisé en ce que**
    le premier équivalent de surface diffère du deuxième équivalent de surface avec chacun des premier et deuxième équivalents de surface calculés pour la même vitesse de rotation.

2. Ensemble selon la revendication 1, dans lequel le premier équivalent de surface différant du deuxième équivalent de surface est fourni par une différence de nombre de disques de séparation tronconiques (92) entre le premier et le deuxième insert de séparation échangeable (6, 6').

3. Ensemble selon la revendication 2, dans lequel les disques de séparation tronconiques (92) du premier insert de séparation échangeable (6) et les disques de séparation tronconiques (92) du deuxième insert de séparation échangeable (6') ont une hauteur totale semblable le long de l'axe (20) de rotation, et dans lequel une distance entre au moins deux des disques de séparation tronconiques (92) du premier insert de séparation échangeable (6) diffère d'une distance entre au moins deux des disques de séparation tronconiques (92) du deuxième insert de séparation échangeable (6').

4. Ensemble selon l'une quelconque des revendications précédentes, dans lequel le premier équivalent de surface différant du deuxième équivalent de surface est fourni par au moins une parmi :

    - une différence de diamètre externe des disques de séparation tronconiques (92) entre le premier et le deuxième insert de séparation échangeable (6, 6'),
    - une différence de diamètre interne des disques de séparation tronconiques (92) entre le premier et le deuxième insert de séparation échangeable (6, 6'), et / ou
    - une différence d'angle $\alpha$ entre un axe (20) de rotation du premier et du deuxième insert de séparation échangeable (6, 6') et une surface interne de l'un des disques de séparation tronconiques (92) entre le premier et le deuxième insert de séparation échangeable (6, 6').

5. Ensemble selon l'une quelconque des revendications précédentes, dans lequel le carter de rotor (82) de chacun du premier et du deuxième insert de séparation échangeable (6, 6') a une première portion d'extrémité axiale (85) et une deuxième portion d'extrémité axiale (87), et dans lequel chacun du premier et du deuxième insert de séparation échangeable (6, 6') comprend au moins un premier raccordement fluidique (94) agencé à la première portion d'extrémité axiale (85).

6. Ensemble selon la revendication 5, dans lequel chacun du premier et du deuxième insert de séparation échangeable (6, 6') comprend au moins un deuxième raccordement fluidique (96) agencé à la deuxième portion d'extrémité axiale (87).

7. Système modulaire de séparateur centrifuge (1) configuré pour séparer un mélange d'alimentation liquide en une phase lourde et une phase légère, le système modulaire de séparateur centrifuge comprenant une unité de base (4) et un premier insert de séparation échangeable (6), dans lequel

    l'unité de base (4) comprend un cadre fixe (8), un élément rotatif (16) configuré pour tourner autour d'un axe (20) de rotation agencé dans le cadre fixe (8), et une unité d'entraînement (18) pour faire tourner l'élément rotatif (16) autour de l'axe (20) de rotation, dans lequel l'élément rotatif (16) délimite un espace interne (26) au moins dans un sens radial, l'espace interne (26) étant configuré pour recevoir au moins une partie du premier insert de séparation échangeable (6) à l'intérieur de celui-ci, **caractérisé en ce que** le système modulaire de séparation (1) comprend un ensemble d'inserts de séparation échangeables (6, 6') selon l'une quelconque des revendications précédentes, dans lequel le premier insert de séparation échangeable (6) forme une partie de l'ensemble d'inserts de séparation échangeables (6, 6').

8. Système modulaire de séparateur centrifuge (1) selon la revendication 7, dans lequel l'élément rotatif (16) a une première extrémité axiale (22) et une deuxième extrémité axiale (24), et l'élément rotatif (16) est doté d'une première ouverture (28) à la première extrémité axiale (22) configurée pour au moins un premier raccordement fluidique (94) du premier ou du deuxième insert de séparation échangeable (6, 6') pour s'étendre à travers la première ouverture (28).

9. Système modulaire de séparateur centrifuge (1) selon la revendication 8, dans lequel l'élément rotatif (16) comprend une deuxième ouverture (30) à la deuxième extrémité axiale (24) configurée pour au moins un deuxième raccordement fluidique (96) du premier ou du deuxième insert de séparation échangeable (6, 6') pour s'étendre à travers la deuxième ouverture (30).

10. Procédé (100) pour faire fonctionner un système modulaire de séparateur centrifuge (1) configuré pour séparer un mélange d'alimentation liquide en une phase lourde et une phase légère selon l'une quelconque des revendications 7 à 9, comprenant les étapes de :

    - fourniture (102) du premier insert de séparation échangeable (6),
    - montage (104) du premier insert de séparation échangeable (6) dans l'espace interne (26) de l'élément rotatif (16),
    - séparation (106) d'un premier lot de mélange d'alimentation liquide dans le système modulaire de séparateur centrifuge (1) en une première phase lourde et une première phase légère en utilisant le premier insert de séparation échangeable,
    - retrait (108) du premier insert de séparation échangeable (6) de l'espace interne (26) de l'élément rotatif (16),

- fourniture (110) du deuxième insert de séparation échangeable (6'),
- montage (112) du deuxième insert de séparation échangeable (6') dans l'espace interne (26) de l'élément rotatif (16),
- séparation (114) d'un deuxième lot du mélange d'alimentation liquide dans le système modulaire de séparateur centrifuge (1) en une deuxième phase lourde et une deuxième phase légère en utilisant le deuxième insert de séparation échangeable (6').

11. Procédé selon la revendication 10, dans lequel chacun du premier et du deuxième lot de mélange d'alimentation liquide comprend une matière solide, et dans lequel le premier lot de mélange d'alimentation liquide diffère du deuxième lot de mélange d'alimentation liquide par au moins un parmi :

- un type de matière solide,
- une concentration de matière solide, et / ou
- un contenu de la première phase légère séparée et un contenu de la deuxième phase légère séparée.

12. Procédé selon la revendication 10 ou 11, dans lequel chacun du premier et du deuxième lot de mélange d'alimentation liquide est un mélange de culture cellulaire.

Fig. 1

**Fig. 2**

**Fig. 3**

Fig. 4

100

| 102 – providing first exchangeable separation insert |
|---|

| 104 – mounting first exchangeable separation insert |
|---|

| 106 - separating first batch of liquid feed mixture |
|---|

| 108 - removing first exchangeable separation insert |
|---|

| 110 - providing second exchangeable separation insert |
|---|

| 112 - mounting second exchangeable separation insert |
|---|

| 114 - separating second batch of liquid feed mixture |
|---|

**Fig. 5**

**EP 3 978 136 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110319248 A
- WO 2015181177 A
- EP 3666394 A
- US 6973925 B

**21**